# EUROPEAN PATENT APPLICATION

(11) **EP 4 324 501 A1**
(43) Date of publication of application: **21.02.2024**
(21) Application number: 22788457.4
(22) Date of filing: 13.04.2022
(51) Int. Cl.: A61M 5/28, A61M 5/315, A61F 9/007, A61M 5/31, A61K 9/00, A61K 9/08, A61K 47/22, A61K 47/26

(54) **PRE-FILLED SYRINGE FILLED WITH LOW-DOSE LIQUID MEDICINE, AND PREPARATION METHOD THEREFOR**

(30) Priority: 15.04.2021 KR 20210049470; 21.06.2021 KR 20210079911
(71) Applicant: Alteogen, Inc, Yuseong-gu Daejeon 34054 (KR)
(72) Inventor: PARK, Soon Jae, Daejeon 34054 (KR); KIM, Kyuwan, Daejeon 34054 (KR); CHU, Seiyoon, Daejeon 34054 (KR)
(74) Representative: Sagittarius IP
(86) International application number: PCT/KR2022/005379
(87) International publication number: WO 2022/220602

(57) **Abstract**

Provided are a syringe, in particular, a pre-filled syringe suitable for injecting liquid pharmaceutical into the eye, and a method of manufacturing the same.

## Description

### TECHNICAL FIELD

The present disclosure relates to a pre-filled syringe filled with low-volume liquid pharmaceutical suitable for injecting liquid pharmaceutical into the eye, and a method of manufacturing the same.

### BACKGROUND ART

A plurality of pharmaceutical is delivered to a patient via syringes. When the pharmaceutical is delivered to the patient through syringes, the contents and the syringes need to be sufficiently sterilized for the safety of the patient and the integrity of the pharmaceutical. In particular, in the case of a pre-filled syringe filled with ophthalmic pharmaceutical, a filling process needs to be performed in a sterile state. In addition, because the combination of consumables that do not come into contact with the pharmaceutical, such as a push rod and a finger hanger, which is a secondary packaging process performed after a process of filling the pharmaceutical, is performed in a non-sterile environment, final sterilization is additionally required. When final sterilization is not performed or when sterilization is not properly performed, there is a risk of endophthalmitis due to contamination of the syringes (Container closure and delivery consideration for intravitreal drug dosing, AAPS PharmSciTech, 2021, doi: 10.1208/s12249-021-01949-4). Therefore, after all of the accessories of the syringes are combined, the contaminants that may remain on the surface of the syringes are sterilized through final sterilization to maintain the safety of the patient and the integrity of the pharmaceutical. In order to maintain the sterility of the finally sterilized syringes until just before use, additional packaging needs to be performed on the outside of the syringes to provide a sterile barrier. According to the International Organization for Standardization (ISO), this is called as aseptic packaging, and aseptic packaging is defined as "the minimum packaging that can prevent the penetration of bacteria and ensure the asepsis of the product at the point of use". According to the ISO, also, final sterilization is defined as "a process in which sterilization is performed in a state in which aseptic packaging is performed" (ISO/TS 11139:2006).

The method of final sterilization as above includes cold gas sterilization, heat sterilization, or irradiation sterilization. However, certain therapeutic agents, such as biological molecular therapeutic agents, are characterized by being vulnerable to low temperature, high temperature, and irradiation. Thus, the final sterilization method for pre-filled syringes filled with biological molecular therapeutic agents is limited, and commercially available sterilizing agents such as ethylene oxide, vaporized hydrogen peroxide, or nitrogen dioxide are used to perform final sterilization. The sterilizing agents as above are used in a vaporized form, and a process of removing the sterilizing agents in a vacuum state is repeated several times after sterilizing agent injection and leaving processes to ensure an appropriate level of sterility, that is, sterility assurance level (SAL: sterility assurance level, governed by ANSI/AAMI ST67).

It was analyzed that the residual amount of the sterilizing agent on the surface after sterilization is completed gradually decreased over time and showed a tendency to decrease 4000 µg/syringe or less that is the standard of ISO 10993-7. However, in the case of ethylene oxide, it may pass through a plastic barrel during the final sterilization process, and degeneration may occur due to the combination of methionine and cystine among amino acids and ethylene oxide (Impact of ethylene oxide sterilization of polymer-based prefilled syringes on chemical degradation of a model therapeutic protein during storage, 2018, J. Pharm. Sci., doi: 10.1016/j.xphs.2018.09.029).

In addition, in the case of vaporized hydrogen peroxide that is a specific sterilizing agent, similar to ethylene oxide, may cause oxidation to amino acids including methionine and cysteine during the final sterilization process (Chemical-gas sterilization of external surface of polymer-based prefilled syringes and its effect on stability of model therapeutic protein, 2021, J. Pharm. Sci., doi: 10.1016/j.xphs.2021.09.003).

In the case of a syringe filled with low-volume pharmaceutical, such as a pre-filled syringe filled with pharmaceutical of 0.1 mL or less, manufactured for intraocular injection, a stopper coupled to a syringe barrel by vacuum pressure during the final sterilization process can be finely moved. In this case, there is a risk that the sterility inside the syringe filled in a sterile environment is destroyed, or liquid pharmaceutical filled in the syringe leak out from the inside of the syringe maintaining the sterility. Thus, there is a need to maintain the sealed state of the syringe while ensuring a level of assurance of sterility.

In addition, in the case of a plastic pre-filled syringe, there is a risk that the remaining amount of the sterilizing agent remaining on the syringe surface during the final sterilization process using the sterilizing agent may pass through the barrel of the plastic syringe and penetrate into the pharmaceutical filled inside the syringe (Chemical-gas sterilization of external surface of polymer-based pre-filled syringes and its effect on stability of model therapeutic protein, 2021, J. Pharm. Sci., doi: 10.1016/j.xphs.2021.09.003).

Finally, even if the airtightness of the syringe as above and the inflow of sterilizing agents in the sterilization process are blocked, in the case of final sterilization performed at room temperature or higher, changes in the quality of pharmaceutical that may occur during the sterilization process need to be carefully controlled through a process cycle design.

In particular, in the case of oxygen, oxygen is dissolved in liquid pharmaceutical in the form of a solution during the sterilization process and causes degeneration of proteins, which are the main components of biological molecular therapeutics (A strategy for the prevention of protein oxidation by drug product in polymer-based syringe. PDA J. Pharm. Sci. and Tech., 2015, doi: 10.5731/pdajpst.2015.01009). Thus, bubble-free filling is required in which bubbles are minimized or bubbles are completely removed in the process of filling the pre-filled syringe with the liquid pharmaceutical.

### DETAILED DESCRIPTION OF THE INVENTION

### TECHNICAL PROBLEM

The present disclosure provides a filling process of a pre-filled syringe that does not cause changes in the composition of liquid pharmaceutical while providing a strong seal for sterilization of the liquid pharmaceutical filled in the syringe and final sterilization for the outside of the syringe.

The present disclosure also provides a method for manufacturing a pre-filled syringe, whereby a pre-filled syringe filled with liquid pharmaceutical and the liquid pharmaceutical in the pre-filled syringe can be maintained in a sterile state, and a prefilled syringe manufactured by using the method.

The present disclosure also provides limiting of the dosing volume of liquid pharmaceutical of a pre-filled syringe to prevent the risk of reusing the pre-filled syringe twice or more.

The present disclosure also provides a pre-filled syringe in which filled liquid pharmaceutical can be stably maintained even under severe conditions such as high temperature.

### TECHNICAL SOLUTION

Hereinafter, the term "nominal maximum filling volume" is a concept that means the maximum filling volume of liquid pharmaceutical that can be stored inside of a barrel 1.

Hereinafter, the term "filling volume" refers to a concept that means the volume of the liquid pharmaceutical actually filled in the barrel 1.

Hereinafter, the term "dosing volume" refers to a concept that means the volume of liquid pharmaceutical injected into a patient. On the other hand, the pre-filled syringe used in the present disclosure is a syringe in which a trace amount of liquid pharmaceutical is stored, and a needle is used in combination with an outlet of the barrel. After the pre-filling of liquid pharmaceutical is performed, a very trace amount of the liquid pharmaceutical is discharged during a priming process to remove bubbles inside of the barrel before the liquid pharmaceutical is dosed to an actual user, and even if a plunger is pressed to the end, due to a tip of the barrel 1 and the accommodation volume of the needle itself connected to the tip, the dosing volume is usually smaller than the filling volume.

Hereinafter, the term "about" may be presented before a specific numerical value. As used herein, the term "about" includes not only an exact number recited after the term, but also a range near or close to the number. Considering the context in which the number is presented, it can be determined whether the number is close to or near the specific number mentioned. In an example, the term "about" may refer to a range from -10% to +10% of a numerical value set forth thereafter. However, the present disclosure is not limited thereto.

### Configuration of pre-filled syringe

A pre-filled syringe according to the present disclosure may include a barrel 1, a stopper 2, and a plunger.

The barrel 1 has a space in which liquid pharmaceutical can be stored, and includes an outlet 1a through which the stored liquid pharmaceutical is discharged, and an inlet 1b that is formed in an opposite direction to a direction of the outlet 1a.

The barrel 1 according to the present disclosure may have a nominal maximum filling volume of from about 0.5 mL to about 1 mL. As will be described later, the liquid pharmaceutical is filled in an inside of the barrel 1 to have a dosing volume of about 0.03 mL to about 0.2 mL, more specifically about 0.04 mL to about 0.195 mL, about 0.05 mL to about 0.19 mL, about 0.06 mL to about 0.185 mL, and about 0.07 mL to about 0.18 mL, more specifically about 0.08 mL to about 0.175 mL, about 0.09 mL to about 0.17 mL, about 0.1 mL to about 0.165 mL, and about 0.11 mL to about 0.16 mL, preferably about 0.12 mL to about 0.155 mL, and more preferably about 0.126 mL to about 0.154 mL.

In the barrel 1 according to the present disclosure, unlike a general syringe, no graduation is formed on the outer surface of the barrel 1, but a priming mark is formed. The priming mark serves to guide the position of a first rib (based on a side close to the liquid pharmaceutical) of the stopper 2 before the use of the pre-filled syringe according to the present disclosure. That is, the priming mark serves as a guide to enable completely removing bubbles inside of the barrel 1 before dosing of the liquid pharmaceutical, and to enable dosing of the liquid pharmaceutical once or more.

A tip 6 may be formed on the outlet 1a of the barrel 1, and a tip cap to be coupled to the tip 6 and a separate cover 7 covering the front of the barrel 1 may be further coupled to the tip 6.

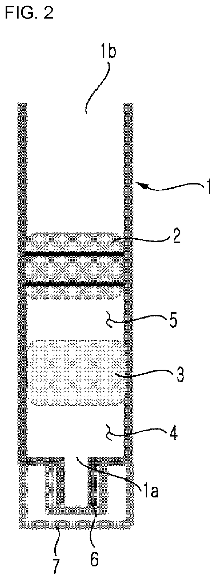

The stopper 2 is disposed in the inner space of the barrel 1. As shown in FIG. 2, the stopper 2 may have a flat contact surface in contact with the liquid pharmaceutical accommodated in the barrel 1, and preferably, the side surface of the stopper 2 is in close contact with the inside of the barrel 1. As will be described later, as the surface in contact with the liquid pharmaceutical has a flat shape, the pressure applied through the plunger may be completely used to discharge the liquid pharmaceutical.

The plunger is connected to the stopper 2 and is configured to apply pressure so that the stopper 2 can be moved in the inner space of the barrel 1. For example, the plunger may extend further to the rear than the barrel 1 (*i.e*., protruding rearward than the barrel 1), so that a user can apply pressure in a direction toward the outlet 1a while holding the protruding portion of the plunger. Thus, the stopper 2 may be moved in a direction toward the outlet 1a, and the liquid pharmaceutical stored in the inner space of the barrel 1 may be discharged to the outside through the outlet 1a. That is, the plunger is configured to vary the internal volume of the barrel 1 between the stopper 2 and the outlet 1a, and serves to discharge the liquid pharmaceutical to the outside.

On the other hand, as the liquid pharmaceutical filled in the barrel 1, a biologic, preferably a protein drug, for example, ophthalmic liquid pharmaceutical containing a fusion protein as an active ingredient, can be used. In an embodiment of the present disclosure, the ophthalmic liquid pharmaceutical may contain aflibercept as a vascular endothelial growth factor antagonist solution.

In the pre-filled syringe of the present disclosure, the barrel 1 is made of a plastic material, and there is no silicone oil inside the barrel 1 and the stopper 2, and when the barrel 1 is filled with the liquid pharmaceutical and the outlet 1a is placed vertically to face down, a shortest distance from the surface of the liquid pharmaceutical to the stopper 2, that is, the height of the "bubbles", is minimized to within about 10 mm so that oxidation and degeneration of the liquid pharmaceutical can be suppressed.

In addition, the pre-filled syringe of the present disclosure does not use silicone oil, which is generally used as a lubricant for reducing the gliding force of the stopper in a glass syringe, and the break-loose force and the gliding force of the stopper of the syringe are less than about 15 N, preferably less than about 10 N, more preferably less than about 5 N, and even more preferably less than about 3 N.

### Method of filling for minimizing bubbles or removing bubbles

In a process of filling the liquid pharmaceutical to the syringe, the air in contact with the liquid pharmaceutical may cause the degeneration of the liquid pharmaceutical. The degeneration of the liquid pharmaceutical may have a fatal effect on the integrity of the therapeutic agent, such as a protein drug.

It is well known that protein drug is vulnerable to oxidation. Oxygen may cause direct oxidation to methionine and cysteine among amino acids that constitute protein, and may produce continuously superoxide radical anion, hydrogen peroxide or hydroxyl radicals, which are reactive oxygen species, due to a trace amount of Fe²⁺ and Cu¹⁺ that remain in the solution (Nonezymatic Cleavage of Proteins by Reactive Oxygen species generated by dithiothreitol and iron, 1985, 260 (29):15394-7).

Because the reactive oxygen species are highly reactive, the reactive oxygen species cause oxidation of methionine and cysteine, and in the case of cysteine, a thiol functional group of a side branch may be gradually oxidized into sulfenic acid (SO), sulfinic acid (SO₂), and sulfonic acid (SO₃), and may also cause decomposition of protein. In particular, in SO₃, irreversible forms of oxidation occur, and reduction does not revert it to the thiol functional group.

Thus, in the process of filling the liquid pharmaceutical in the syringe, the bubbles need to be completely removed, or the remaining bubbles need to be minimized so as to maintain the integrity of the liquid pharmaceutical and to suppress the degeneration of the liquid pharmaceutical until the expiration date.

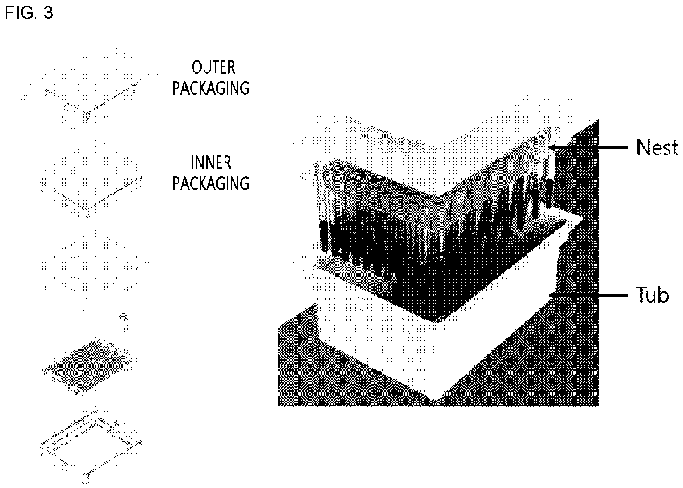

The filling process of the pre-filled syringe that minimizes the bubbles is as follows (see FIG. 3).

The sterilized barrel 1 with outer packaging is moved into an aseptic filling chamber, and the outer packaging of the sterilized pre-filled syringe is removed. Subsequently, inner packaging of the sterilized barrel 1 is removed.

In a tub in which the inner packaging of the sterilized barrel 1 is removed, the sterilized pre-filled syringe is fixed to a nest. A robot arm in the aseptic filling chamber transfers a syringe to a filling position so as to fill the liquid pharmaceutical inside the barrel 1. When filling is completed, the stopper 2 is combined with the inside of the barrel 1 so as to maintain sterility. The syringe that has been combined with the stopper 2 is moved back to the nest and fixed to its initial position. When filling is completed in all syringes in the tub, inner packaging is performed again in a sterile chamber, and a packaging material including the syringe of which inner packaging is completed, is moved to the outside of the sterile chamber.

Hereinafter, a filling process in which the bubbles are minimized inside the barrel 1 will be described in detail.

In this regard, a method of combining a stopper by applying a vacuum inside the barrel 1 in the syringe filling process has been used. However, in the case of ophthalmic liquid pharmaceutical, for the reason that intraocular pressure increases as the dose of the liquid pharmaceutical increases, the dosing volume of the syringe is limited to 0.02 mL to 0.05 mL, and an actual filling volume is limited to 0.1 mL to prevent reuse and two - time usages of the syringe. In this case, when filling of a small amount of liquid pharmaceutical is performed, the vacuum inside the barrel 1 is weak so that the stopper 2 cannot provide sufficient pressure required to be moved to the surface of the liquid pharmaceutical. In contrast, when a vacuum of sufficient pressure is provided to the barrel 1 so as to move the stopper 2 to the surface of the liquid pharmaceutical, the liquid pharmaceutical filled in the barrel 1 rises and is moved along the barrel 1 by pressure, and in this procedure, a phenomenon (boiling) in which the liquid pharmaceutical is boiling, a phenomenon in which the liquid pharmaceutical is scattered inside the barrel 1, and a phenomenon in which some of the liquid pharmaceutical form a ring, that may ascend through the wall of the barrel 1. In this case, all of the phenomena may affect the integrity of the liquid pharmaceutical. In order to solve the problem when the filling of the small amount of liquid pharmaceutical is performed, there is a method of reducing the movement distance of the stopper 2 by reducing the length of the syringe and filling with low pressure. In this case, however, the syringe needs to be custom-manufactured, and additional tasks and costs are generated in accordance with the license, which affects the overall pharmaceutical development period and price.

In order to minimize the remaining bubbles in the barrel 1 during the syringe filling process, the present inventors have used the following methods.

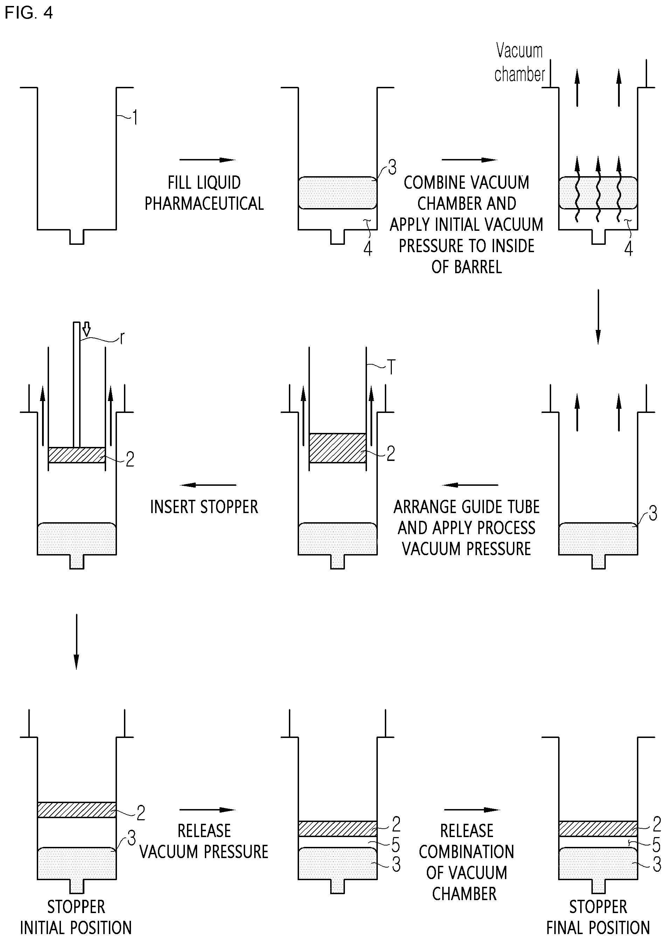

First, liquid pharmaceutical 3 was filled inside the barrel 1. In the present disclosure, trace amounts of liquid pharmaceutical 3 of about 0.05 mL to about 0.25 mL, about 0.06 mL to about 0.24 mL, about 0.07 mL to about 0.23 mL, about 0.08 mL to about 0.22 mL, about 0.09 mL to about 0.21 mL, and about 0.1 mL to about 0.2 mL, more specifically about 0.11 mL to about 0.17 mL, and more specifically about 0.112 mL to about 0.168 mL may be filled to have the dosing volume of about 0.03 mL to about 0.2 mL, and a space 4 is formed between the outlet 1a and the liquid pharmaceutical 3 based on the fact that the filled liquid pharmaceutical 3 have high viscosity and concentration (see FIG. 4).

Next, after the outlet 1a of the barrel 1, in which the liquid pharmaceutical 3 was filled, was positioned in a vertical downward direction, the vacuum chamber was combined with the inlet 1b, and the inside of the barrel 1 was made to be in a vacuum state.

Next, an initial vacuum pressure was applied to the inside of the barrel 1 so that the air located in the space 4 between the outlet 1a and the liquid pharmaceutical 3 was moved to the inlet 1b and thus, the space 4 between the outlet 1a and the liquid pharmaceutical 3 was removed or minimized.

After the space (bubbles) 4 between the outlet 1a and the liquid pharmaceutical 3 was removed, a process vacuum pressure lower than the initial vacuum pressure was applied, and a guide tube t in which the stopper 2 was installed was inserted into the barrel 1.

Next, a rod **r** was inserted into the rear of the guide tube **t,** and the stopper 2 was pushed into the barrel 1. The position of the stopper 2 at this time is defined as an "initial position". The initial position is a position at which the shortest distance between the surface of the liquid pharmaceutical 3 and the stopper 2 is less than the shortest distance between the inlet 1b and the stopper 2.

Next, the process vacuum pressure applied to the inside of the barrel 1 was released. When the process vacuum pressure is released, pressure outside the barrel 1 is higher than pressure inside the barrel 1, so that the stopper 2 is lowered along the barrel 1 by a pressure difference.

Next, the vacuum chamber combined with the barrel 1 was removed to complete the pre-filling process of the syringe.

That is, as a way to solve the problem in small amounts of liquid pharmaceutical filling, the present inventors had filled by placing the initial position of the stopper 2 closer to the surface of the liquid pharmaceutical than the inlet 1b of the barrel 1 and by applying two different vacuum pressures (initial vacuum pressure and process vacuum pressure). Through such a filling method, the space between the liquid pharmaceutical and the outlet 1a is removed, and the height of a space 5 between the surface of the liquid pharmaceutical and the stopper 2 is about 10 mm, preferably about 5 mm, more preferably about 4 mm, more preferably about 3 mm, more preferably about 2 mm, and more preferably about 1 mm or less. The height of the surface of the liquid pharmaceutical and the stopper 2 measured in an embodiment of the present disclosure was about 3 mm. In addition, through such a filling method, the space between the liquid pharmaceutical and the outlet 1a after the liquid pharmaceutical filling is removed, and the surface area of the space 5 located between the liquid pharmaceutical and the stopper 2 is about 180 mm² or less, preferably about 110 mm² or less, more preferably about 95 mm² or less, more preferably about 85 mm² or less, more preferably about 65 mm² or less, and more preferably about 50 mm² or less. The volume of the space 5 between the liquid pharmaceutical and the stopper 2 measured in an embodiment of the present disclosure is 0 mL to about 0.9 mL, specifically about 0.3 mL to about 0.9 mL and 0 mL to about 0.8 mL, specifically about 0.4 mL to about 0.8 mL and 0 mL to about 0.7 mL, specifically about 0.5 mL to about 0.7 mL and 0 mL to about 0.6 mL, specifically about 0.5 mL to about 0.6 mL, more specifically about 0.555 mL to about 0.59 mL, and most preferably 0 mL. Through the above method, the volume of the bubbles (space) in contact with the liquid pharmaceutical in the pre-filled syringe can be minimized. Thus, the oxidation and degeneration of the liquid pharmaceutical due to oxygen can be reduced.

### Syringe pressure

The pressure of a syringe needs to be secured for the convenience of use. In the case of an existing glass syringe, typically, convenience is increased by applying silicone oil to the inside of the barrel. Thus, a force required to move the stopper is reduced. However, in the case of a glass syringe, even when silicone oil is applied to the glass syringe, a stopper break-loose force is relatively high compared to a stopper gliding force (Minimizing oxidation of freeze-dried monoclonal antibodies in polymeric vials using a smart packaging approach, 2021, doi: 10.3390)/pharmaceutics13101695).

When an excessive force is required to move the stopper, problems may arise in use. For example, when a significant force is required in order to dose liquid pharmaceutical, accurate dosing volume settings or smooth dosing may become more difficult.

In addition, when a significant force is required when dosing the liquid pharmaceutical, the pressure at the time of injection is high, even though the stopper needs to stop the dosing by contacting the outlet of the syringe so that some users cannot easily recognize this so that an overdose may occur. In particular, in the case of ophthalmic liquid pharmaceutical, because the protein, which is a major component of the liquid pharmaceutical, is concentrated to a high concentration in order to reduce the dose, when the stopper is further moved finely, it may lead to an overdose compared to liquid pharmaceutical with a low concentration. Due to smooth dosing, the risk of local tissue damage caused by the movement of the syringe while dosing can be reduced. The break-loose force and the gliding force of pre-filled syringes known in the art to which the present disclosure pertains are typically in the range of less than 20 N.

In the case of pre-filled syringes made of glass, generally, silicone oil is used to reduce the stopper break-loose force. In this case, particle generation of the liquid pharmaceutical is accelerated, and aggregation increases, which affects the stability and integrity of the liquid pharmaceutical (Effect of the siliconization method on particle generation in a monoclonal antibody formulation in pre-filled syringes. J. Pharm. Sci. 2015, doi: 10.1002/jps.24387).

Visible particles were confirmed when a glass syringe coated with about 250 µg of silicone oil inside the barrel was stored at room temperature (25°C ± 2°C) for 6 months. On the other hand, it was analyzed that in a plastic syringe, particles that can be visually confirmed under the same conditions were not found until 12 months of storage, and sub-visible particles were also found to have no difference from the measured values at the start of storage.

In order to overcome the problem of the syringe made of glass, a syringe made of a plastic material, preferably a cyclic olefin copolymer (COC) or a cyclic olefin polymer (COP) material, may be used. Compared to the glass syringe, the plastic syringe can provide a stopper releasing force and gliding force that is convenient for use without applying silicone oil to the inside of the barrel. In addition, problems such as a decrease in convenience caused by the difference between the gliding force and the stopper releasing force caused by applying a trace amount (1 µg to 100 µg) of silicone oil to the glass syringe were also corrected. In the present embodiment, the stopper releasing force of the glass pre-filled syringe having a maximum filling volume of 1 mL and coated with 1 µg to 100 µg of silicone oil was about 8 N, and the gliding force was about 6 N. In contrast, in the present embodiment, the stopper releasing force of the pre-filled syringe having a maximum filling volume of 0.5 mL and no silicone oil applied to the inside of the barrel was about 2 N, and the gliding force was also about 2 N.

Thus, the stopper releasing force of the glass pre-filled syringe coated with silicone oil is higher than the gliding force, and the user's convenience is low, and some users may have problems due to the high gliding force. On the other hand, in the case of a pre-filled syringe made of COP plastic, the stopper break-loose force and the stopper gliding force are similar to each other, and the liquid pharmaceutical can be dosed with a lower force compared to glass, and when the stopper is in contact with the entrance of the outlet, the user can easily detect this so that the risk of overdose can be relatively low.

### Filling volume

As described above, in order to minimize or completely remove oxygen in contact with the liquid pharmaceutical when filling the liquid pharmaceutical, a certain amount of the liquid pharmaceutical needs to be filled inside the barrel 1. In this case, when the liquid pharmaceutical is filled with more than two-time usages, a syringe with inherent risks of contamination and cross-infection due to reuse is manufactured. In particular, in the case of ophthalmic liquid pharmaceutical, it is important to keep the dose low because intraocular pressure increases when the dose of the liquid pharmaceutical is injected 0.05 mL or more. For this purpose, it is preferable to fill only the minimum amount of liquid pharmaceutical required to remove bubbles in the syringe. In the case of commercially available ophthalmic liquid pharmaceutical for macular degeneration, a single dose is about 0.05 mL, and the actual filling volume for bubble removal is about 0.165 mL. By using a pre-filled syringe filled with ophthalmic liquid pharmaceutical for macular degeneration having a commercialized maximum nominal filling volume of 1 mL, the bubbles were removed according to the United States Pharmacopoeia (USP <697>, Container content for Injection), and as a result of measuring the remaining amount, the volume of the remaining liquid pharmaceutical was about 0.121 mL to about 0.132 mL. This result means that the risk of double dosing is inherent as the remaining amount of 0.100 mL or more, which is two-time usages, can be secured even after removing the bubbles.

In order to solve this problem, a syringe having a small inner diameter is used in the present disclosure. Specifically, the inner diameter of the syringe according to the present disclosure may be about 3 mm to about 8 mm, specifically about 4 mm to about 7 mm, and more specifically about 4.5 mm to about 6.5 mm. In this case, the distance of the stopper to be moved to remove the bubbles is the same as for a syringe with a large inner diameter or a syringe having a small inner diameter, but the volume inside the barrel reduced by moving the stopper for removing bubble is smaller for a syringe with a small inner diameter compared to a syringe with a large inner diameter.

By applying this principle, when about 0.112 mL to about 0.168 mL is theoretically filled in a syringe with a small inner diameter, a pre-filled syringe filled with only one dose or more and less than two-time usages can be manufactured after bubble removal. In this case, compared to a syringe that can be dosed twice, the syringe is characterized in that the patient's stability is secured. In the present embodiment, a pre-filled syringe with a maximum nominal filling volume of about 0.5 mL is filled with the pharmaceutical to a lower limit of a filling amount of about 0.115 mL, the bubbles are removed and the volume measured by recovering the remaining drug according to the United States Pharmacopoeia (USP Container content for Injection), was about 0.069 mL to about 0.074 mL. This is a filling amount that eliminates the risk of two-time usages.

As a result, when the filling volume of the liquid pharmaceutical filled inside the barrel 1 is adjusted to about 0.11 mL to 0.17 mL, a syringe that eliminates the risk of contamination and cross-infection due to the two-time usages can be used.

### Acceleration stability

In order to solve the problem that the glass pre-filled syringe causes the generation of particles of liquid pharmaceutical and the increase in aggregates due to the application of silicone oil, a pre-filled syringe made of a COC or COP material may be used in the present disclosure.

The plastic pre-filled syringe has the advantage of securing user convenience without applying silicone oil, but has the disadvantage that the gas permeability is higher than that of the glass syringe, and may affect the stability of the liquid pharmaceutical caused by a material leaking from the material for the plastic syringe.

In an embodiment of the present disclosure, a glass syringe with silicone oil of about 250 µg/syringe applied, a COC syringe with no silicone oil applied, and a COP syringe with no silicone oil applied were stored at room temperature (25°C ± 2°C) for 12 months to evaluate the increase in particles and the increase in impurities (aggregate). The liquid pharmaceutical filled in the syringe are liquid pharmaceutical according to Example 1. In addition, filling each syringe with liquid pharmaceutical was performed by way of the method according to Example 2.

At the time of 4 months of the test, particles were visually confirmed in the liquid pharmaceutical in the syringe coated with silicone oil, and the number of particles was too large for sub-visible particles to be measured by using experimental equipment. In contrast, in the case of the liquid pharmaceutical stored in a plastic syringe made of a COC and COP material, the protein concentration, particles that can be visually confirmed, and the content of particles and impurities that cannot be visually confirmed using experimental equipment were analyzed similarly to initial ones.

Thus, it can be noted that a plastic pre-filled syringe is more suitable for maintaining the safety and integrity of the liquid pharmaceutical filled by way of the method of the present disclosure than a glass pre-filled syringe coated with silicone oil.

According to an aspect of the present disclosure, there is provided a pre-filled syringe including a hollow barrel having an outlet through which liquid pharmaceutical is discharged, and an inlet in an opposite direction to a direction of the outlet, a stopper disposed inside the barrel, and a plunger capable of being in contact with the stopper and moving the stopper toward the outlet to vary an internal volume of the barrel between the stopper and the outlet, wherein the barrel has a nominal maximum filling volume of about 0.5 mL to about 1 mL, and the liquid pharmaceutical is filled inside the barrel so that the barrel has a dosing volume of about 0.03 mL to about 0.2 mL, and after filling the liquid pharmaceutical into the barrel, a space is formed between the stopper from the surface of the liquid pharmaceutical, and the volume of the space is 0 mL to about 0.9 mL.

The volume of the space may be 0 mL to about 0.6 mL.

The inside of the barrel may be filled with about 0.11 mL to about 0.17 mL of the liquid pharmaceutical.

A distance from the surface of the liquid pharmaceutical, which is the height of the space, to the stopper may be about 10 mm or less, specifically about 5 mm or less, and more specifically about 4 mm or less, about 3.8 mm or less, or about 3.5 mm or less. Furthermore, the surface area of the space is about 180 mm² or less, preferably about 110 mm² or less, more preferably about 95 mm² or less, still more preferably about 85 mm² or less, even more preferably about 65 mm² or less, more preferably about 50 mm² or less, and most preferably about 50 mm² to about 0 mm².

The barrel may have an inner diameter of about 3 mm to about 8 mm.

The liquid pharmaceutical may be filled in the barrel so that the liquid pharmaceutical has a dosing volume of about 0.126 mL to about 0.154 mL.

The liquid pharmaceutical may be liquid pharmaceutical containing a vascular endothelial growth factor (VEGF) antagonist solution.

The liquid pharmaceutical containing the vascular endothelial growth factor antagonist solution may have a concentration of about 1 mg/mL to about 300 mg/mL, specifically a concentration of about 1 mg/mL to about 150 mg/mL, more specifically about 10 mg/mL to about 100 mg/mL concentration, and more specifically about 20 mg/mL to about 50 mg/mL concentration. The liquid pharmaceutical may include aflibercept, ranibizumab, bevacizumab, and the like, and is preferably liquid pharmaceutical for ocular injection.

The liquid pharmaceutical containing the vascular endothelial growth factor antagonist solution may have a specific gravity of about 1 to about 1.1, specifically about 1.01 to about 1.05, and more specifically about 1.038.

The liquid pharmaceutical containing the vascular endothelial growth factor antagonist solution may have a viscosity of about 3 cP to about 4 cP, specifically about 3.2 cP to about 3.6 cP, and more specifically about 3.46 cP.

The liquid pharmaceutical containing the vascular endothelial growth factor antagonist solution may have about pH 5 to about pH 8, and a buffering solution containing histidine, tris, bis-tris, phosphate, acetic acid, succinic acid, or the like as a buffering solution exhibiting an effect at corresponding pH may be contained in the liquid pharmaceutical, and preferably, a buffering solution containing histidine may be used. In addition, the liquid pharmaceutical may include a surfactant, and polysorbate, poloxamer, etc. may be used as the surfactant, and preferably 0% (w/v) to 0.1% (w/v) polysorbate 20, polysorbate 80 or poloxamer may be used. In addition, the liquid pharmaceutical may include any one or more selected from the group consisting of 2.5% to 10% sucrose, trehalose, mannitol, and glucose as a stabilizer.

The barrel may be made of plastic, and silicone oil may not be applied therein.

The barrel may be made of COC (Cyclic Olefin Copolymer) or COP (Cyclic Olefin Copolymer).

The stopper break-loose force may be less than about 11 N, specifically less than about 7 N, and more specifically less than about 4 N, less than about 3 N, or about 2 N.

The stopper gliding force may be less than about 11 N, specifically less than about 7 N, and more specifically less than about 4 N, less than about 3 N, or about 2 N.

The stopper break-loose force and/or the stopper gliding force may be significantly reduced so that injection can be performed smoothly without applying force.

The pre-filled syringe may use at least one selected from NO₂, H₂O₂, and EtO gas, preferably NO₂ gas, as a final sterilization method.

According to another aspect of the present disclosure, there is provided a pre-filled syringe including a hollow barrel having an outlet through which liquid pharmaceutical is discharged, and an inlet in an opposite direction to a direction of the outlet, a stopper disposed inside the barrel, and a plunger capable of being in contact with the stopper and moving the stopper toward the outlet to vary an internal volume of the barrel between the stopper and the outlet, wherein the pre-filled syringe is manufactured by performing operations of filling liquid pharmaceutical inside of the barrel; combining a vacuum chamber with the barrel and applying an initial vacuum pressure to the inside barrel; applying a process vacuum pressure lower than the initial vacuum pressure to the inside of the barrel and disposing the stopper inside the barrel, and releasing the process vacuum pressure applied to the inside of the barrel.

After the filling of the liquid pharmaceutical inside of the barrel, the operations may further include forming a first space between the outlet and the liquid pharmaceutical.

After the applying of the initial vacuum pressure to the inside of the barrel, the operations may further include moving air in the first space toward the inlet.

After the disposing of the stopper inside the barrel, the operations may further include forming a second space between the stopper and the liquid pharmaceutical, wherein the height of the second space is less than the height between the stopper and the inlet.

After the releasing of the process vacuum pressure applied to the inside of the barrel, the operations may further include moving the stopper up to a certain position toward the liquid pharmaceutical.

The barrel may have a nominal maximum fill volume of about 0.5 mL to about 1 mL, and may be filled with liquid pharmaceutical inside the barrel to have a dosing volume of about 0.03 mL to about 0.2 mL, and after the moving of the stopper to a predetermined position toward the liquid pharmaceutical, the volume of the space formed between the liquid pharmaceutical and the stopper may be 0 mL to about 0.9 mL.

According to another aspect of the present disclosure, there is provided a pre-filling method of a pre-filled syringe, the pre-filled syringe including a hollow barrel having an outlet through which liquid pharmaceutical is discharged, and an inlet in an opposite direction to a direction of the outlet, a stopper disposed inside the barrel, and a plunger capable of being in contact with the stopper and moving the stopper toward the outlet to vary an internal volume of the barrel between the stopper and the outlet, the pre-filling method including: filling liquid pharmaceutical inside of the barrel, *i.e*., forming a first space between the outlet and the liquid pharmaceutical, combining a vacuum chamber with the barrel and applying an initial vacuum pressure to the inside of the barrel so that air in the first space is moved toward the inlet; disposing the stopper inside the barrel, *i.e*., forming a second space between the stopper and the liquid pharmaceutical, wherein a height of the second space is less than a height between the stopper and the inlet; and releasing a process vacuum pressure applied to the inside of the barrel so that the stopper is moved up to a certain position toward the liquid pharmaceutical.

### EFFECTS OF THE INVENTION

According to the present disclosure, in a pre-filled syringe according to the present disclosure, filled liquid pharmaceutical can be stably maintained even during long-term storage at a high temperature.

According to the present disclosure, one-time low-volume liquid pharmaceutical is filled in the pre-filled syringe so that two-time usages can be prevented and thus contamination caused by reuse can be prevented.

According to the present disclosure, a stopper break-loose force and/or stopper gliding force are significantly reduced so that injection can be performed smoothly without applying force.

According to the present disclosure, the volume of bubbles in the liquid pharmaceutical is limited so that the bubbles can be easily removed or a bubble removal operation can be omitted and thus convenience of use can be enhanced.

According to the present disclosure, the volume of bubbles in the liquid pharmaceutical is limited so that oxidation of the liquid pharmaceutical caused by oxygen contained in the bubbles is minimized and thus the stability of pharmaceutical can be increased.

According to the present disclosure, when the liquid pharmaceutical is filled, the liquid pharmaceutical is filled by adjusting an initial vacuum pressure and/or a process vacuum pressure so that loss of pharmaceutical is minimized and simultaneously the volume of the bubbles can be controlled after filling.

### DESCRIPTION OF THE DRAWINGS

FIG. 1 is a view for describing the term "space (bubbles)" used in the present disclosure;
FIG. 2 is a view for describing two spaces (a first space and a second space) generated when liquid pharmaceutical is filled inside of a barrel;
FIG. 3 is a view for describing configurations used to manufacture a pre-filled syringe;
FIG. 4 is a view for describing a method of manufacturing a pre-filled syringe according to an embodiment of the present disclosure;

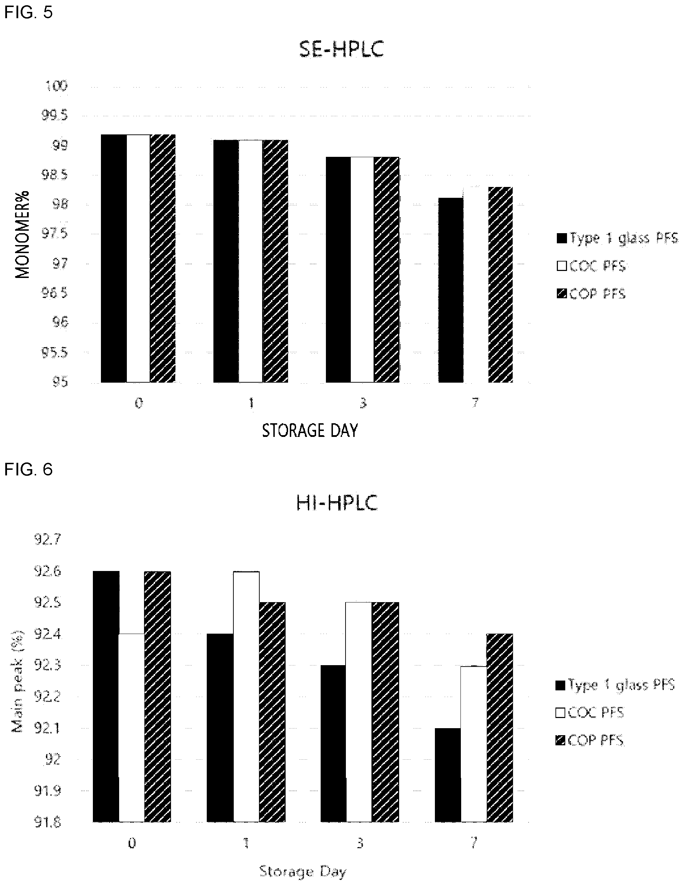

FIG. 5 is a graph showing the results of severe stability SE-HPLC analysis for three types of nominal maximum filling volume 0.5 mL pre-filled syringes; and
FIG. 6 is a graph showing the results of severe stability HI-HPLC analysis for three types of nominal maximum filling volume 0.5 mL pre-filled syringes.

### MODE OF THE INVENTION

Hereinafter, the configuration of the present disclosure will be described in detail by describing specific embodiments. However, it is obvious to one skilled in the art to which the present disclosure pertains that the scope of the present disclosure is not limited to the description of the embodiments.

### <Example 1: Preparation of liquid pharmaceutical>

Liquid pharmaceutical was prepared so that the final concentration of recombinant aflibercept was 40 mg/mL. The liquid pharmaceutical has a pH of 6.0 and contains 5% sucrose and 0.01% (w/v) polysorbate 20 in a 10 mM histidine buffering solution.

The specific gravity of the liquid pharmaceutical was 1.038, and the viscosity was 3.46 cP.

In the following embodiments, the liquid pharmaceutical prepared as above was used.

### <Example 2: Filling of liquid pharmaceutical>

In the present embodiment, a process for minimizing the bubble volume when filling a pre-filled syringe with liquid pharmaceutical is presented.

First, the inside of a barrel 1 was filled with the liquid pharmaceutical. After the liquid pharmaceutical was filled, a first space was formed between an outlet 1a and the liquid pharmaceutical.

Next, after positioning the outlet 1a of the barrel 1 of the syringe vertically downward, a vacuum chamber was coupled to an upper end of the barrel 1, that is, the inlet 1b in the opposite direction to the outlet 1a. The air inside the barrel 1 was removed through the vacuum chamber to make the inside of the barrel to be in a vacuum state.

Next, an initial vacuum pressure was applied to the barrel 1 to move the air located in the first space in the direction of the inlet 1b. Through this process, bubbles between the liquid pharmaceutical, and the outlet 1a were removed or minimized.

After the air located in the first space was removed, the initial vacuum pressure applied to the barrel 1 was changed to a process vacuum pressure. Here, the process vacuum pressure is a pressure having a value lower than the initial vacuum pressure. While the initial vacuum pressure is a pressure applied to exhaust the air located in the first space, the process vacuum pressure is a pressure that allows a vacuum to be maintained inside the barrel 1, and when too large a value of the process vacuum pressure is applied to the barrel 1, it is preferable that the process vacuum pressure is lower than the initial vacuum pressure because a boiling phenomenon occurs in which the liquid pharmaceutical filled inside of the barrel 1 rises along the inner wall of the barrel 1.

Next, a stopper 2 was positioned inside a guide tube **t** provided therein, and the stopper 2 was positioned inside the barrel 1 through a rod **r.** The position of the stopper 2 at this time is defined as an "initial position", and in the initial position, the shortest distance between the stopper 2 and the surface of the liquid pharmaceutical, is less than the shortest distance between the stopper 2 and the inlet 1b.

Next, the process vacuum pressure being applied to the barrel 1 was released. The pressure between the stopper 2 and the liquid pharmaceutical is close to bacuum, whereas the pressure outside the stopper 2 is higher than the vacuum so that the stopper 2 naturally descended toward the liquid pharmaceutical due to a pressure difference.

Thereafter, the vacuum chamber was removed from the barrel 1 to complete the filling process.

On the other hand, the strength of each vacuum pressure (initial vacuum pressure and process vacuum pressure) applied to the barrel 1 may be otherwise determined depending on the type of liquid pharmaceutical filled in the barrel 1 and the sensitivity to vacuum of the liquid pharmaceutical.

The volume of the space positioned between the stopper 2 and the liquid pharmaceutical of the pre-filled syringe obtained through this process is about 0.9 mL or less, preferably about 0.8 mL or less, and more preferably about 0.7 mL or less, about 0.6 mL or less, about 0.5 mL or less, about 0.4 mL or less, and about 0.3 mL to 0 mL.

In the present disclosure, a vacuum chamber may be installed at the inlet 1b in an opposite direction to the outlet 1a to make the inside of the barrel 1 in a vacuum state, and then an initial vacuum pressure may be applied to the barrel 1 so that the bubbles present on the outlet 1a of the barrel 1 can be moved between the liquid pharmaceutical and the stopper 2 in an opposite direction to the outlet 1a of the syringe, the guide tube **t** may be placed inside the barrel while the process vacuum pressure is being applied, and then the stopper 2 may be pushed from the guide tube **t** to the inside of the barrel 1 so that the stopper can be moved to the initial position, and the process vacuum pressure can be released to move the stopper 2 closer to the surface of the liquid pharmaceutical so that bubbles can be minimized or eliminated. When such a method is not employed, boiling of the liquid pharmaceutical (boiling by vacuum) occur, or a phenomenon that the liquid pharmaceutical splashing in all directions in the barrel 1 occurs, which has a great effect on the stability of the liquid pharmaceutical.

### <Example 3: Bubble height measurement>

In the present embodiment, the bubble height of a syringe filled to about 0.115 mL, similar to the lower limit of the filling volume, was measured. Thus, it was evaluated whether the bubbles were minimized by vacuum filling. An identification number was assigned to each syringe sample.

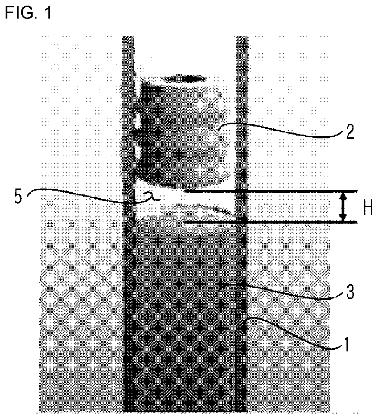

The syringe was horizontally positioned by placing the outlet 1a of the barrel 1 of the syringe in a ground direction, and the barrel 1 was gently tapped so that the bubbles in the pre-filled syringe were moved between the liquid pharmaceutical and the stopper in an opposite direction to a direction of the outlet 1a in an axial direction. As shown in FIG. 1A, the shortest distance from the surface of the liquid pharmaceutical to the stopper 2 was measured using a caliper.

Table 1 below shows the results of measuring the bubble height after filling of a plastic pre-filled syringe with a maximum nominal filling volume of about 0.5 mL. The liquid pharmaceutical prepared as in Example 1 was used, and a target filling volume was about 0.115 mL.

The bubble height of 10 pre-filled syringes pre-filled with the liquid pharmaceutical of Example 1 of the present disclosure by using the method of Example 2 was about 3.2 mm to about 3.4 mm, with an average of about 3.3 mm, and a standard deviation of about 0.1 mm.

When filling by using the method of Example 2, the bubble height could be minimized to about 3 mm.

In addition, as a result of measuring the volume of the bubble, it was measured to be about 0.555 mL to about 0.59 mL, and it was confirmed that the average was about 0.572 mL and the standard deviation was about 0.011 mL.

That is, in the present embodiment, the volume of the bubbles was also minimized, and it was confirmed that contact between the liquid pharmaceutical pre-filled inside the barrel 1 and the air can be minimized to prevent degeneration of the liquid pharmaceutical.

**[Table 1]**

| Example 1 | Syringe No | | | | | | | | | | Mean | SD |
|---|---|---|---|---|---|---|---|---|---|---|---|---|
| | #1 | #2 | #3 | #4 | #5 | #6 | #7 | #8 | #9 | #10 | | |
| Measured Bubble Height (mm) | 3.2 | 3.3 | 3.3 | 3.4 | 3.3 | 3.4 | 3.3 | 3.4 | 3.3 | 3.3 | 3.3 | 0.1 |
| Calculated Bubble Surface Area (mm2) | 81.9 | 83.4 | 83.4 | 84.9 | 83.4 | 84.9 | 83.4 | 84.9 | 83.4 | 83.4 | 83.7 | 0.9 |
| Calculated Bubble Volume (mL) | 0.555 | 0.572 | 0.572 | 0.59 | 0.572 | 0.59 | 0.572 | 0.59 | 0.572 | 0.572 | 0.572 | 0.011 |

### <Example 4: Measurement of filling volume of liquid pharmaceutical>

In the present embodiment, it was evaluated whether practical use is possible after filling the pre-filled syringe with a filling volume of about 0.112 mL to about 0.168 mL. The liquid pharmaceutical prepared as in Example 1 were used.

Filling was performed inside a sterilization process chamber under sterile conditions (grade A). The pre-filled syringes before filling after pre-sterilization were completed were received in the sterilization process chamber in an outer packaging state. Inside the sterilization process chamber, the outer packaging was removed using automated equipment, and a Tyvec^{®} sheet used as the inner packaging was removed using the automated equipment. One pre-filled syringe fixed at 10×10 in a nest in the tub was moved to the filling position using a robot arm. When the movement was finished, the zero point of a scale connected to the robot arm holding the barrel 1 of the pre-filled syringe was set. Each pre-filled syringe was weighed after filling while filling was performed. After the measurement was completed, the coupling operation of the stopper 2 was performed. The pre-filled syringe, which was filled with the liquid pharmaceutical and coupled to the stopper 2, returned to the original position of the nest and fixed to the nest. The Tyvec^{®} sheet used for inner packaging in the sterilization chamber was sealed using the automated equipment. The sealed tub was transferred out of the sterilization chamber.

The number of pre-filled syringes filled with the liquid pharmaceutical used for the measurement was 5,995. The table below shows the mean and standard deviation of filling results for pre-filled syringes.

The average weight of the pharmaceutical filled in 5,995 syringes was about 0.119 g, which was analyzed to be about 0.115 mL ± 0.0018 mL when converted to the volume considering the specific gravity.

**[Table 2]**

| Process factor name | Unit | Reference value (specific gravity: 1.038) | Reference range | Average | Standard deviation |
|---|---|---|---|---|---|
| Filling volume | gram (g) | 0.119 | 0.107 - 0.131 | 0.119 | 0.0018 |

### <Example 5: Measurement of actual capacity>

In the present embodiment, the actual volume was measured after removing the bubbles of the syringe filled with about 0.115 mL similar to the lower limit of the filling volume by using the method of Example 2. Thus, it was evaluated whether the actual capacity was greater than or equal to the dose even when filling with the lower limit of the filling amount. A test method was referred to the US Pharmacopoeia (USP <697>, Container content for Injection). A weighing dish suitable for measuring the actual capacity weight of the prepared sample was placed on the scale and the zero point was adjusted. The plunger was connected to the stopper 2 in the barrel of the recovered prefilled syringe. A finger hook was coupled to the barrel 1 to fix a plunger rod so as not to come off. A luer lock tip cap protecting the outlet 1a was removed, and a 30G needle was attached. An identification number was assigned to each syringe sample. With the outlet 1a of the syringe facing upward, the bubbles in the pre-filled syringe were moved to the outlet 1a by gently tapping the barrel 1. It was confirmed that the bubbles were removed by pushing the plunger and a reagent was condensed on a needle. The contents filled in each sample were extracted with the weighing dish by slowly pressing the plunger rod with a constant force. The weight was recorded for each identification number of each pre-filled syringe. The actual volume was calculated by dividing the measured weight by the specific gravity (specific gravity of the liquid pharmaceutical was 1.038/1 mL = 1.038 g).

The table below shows the actual capacity measurement results of the pre-filled syringe. Table 3 shows the results of measuring the actual volume after filling about 0.115 mL of the liquid pharmaceutical into a plastic pre-filled syringe with a maximum nominal filling volume of about 0.5 mL. Table 4 shows a pre-filled syringe filled with a commercialized macular degeneration agent, a glass syringe with a maximum nominal filling volume of about 1.0 mL, and a filling volume of about 0.165 mL before bubble removal.

As shown in the results below, after removing the bubbles of the plastic syringe of about 0.5 mL, the dosing volume is about 0.070 mL and thus, two-time usages are not possible. On the other hand, in the case of a syringe made of about 1.0 mL glass material, after removing the bubble, the dosing volume is about 0.127 mL and thus, two-time usages are possible.

**[Table 3]**

| | Syringe No. | | | | | Mean | SD | CV |
|---|---|---|---|---|---|---|---|---|
| | #1 | #2 | #3 | #4 | #5 | | | |
| Measured weight (mg) | 72.48 | 71.92 | 69.58 | 77.06 | 75.15 | 73.24 | 2.91 | 4.0 |
| Calculated volume (mL) | 69.83 | 69.29 | 67.03 | 74.24 | 72.40 | 70.56 | 2.81 | 4.0 |

**[Table 4]**

| | Syringe No. | | | | | Mean | SD | CV |
|---|---|---|---|---|---|---|---|---|
| | #1 | #2 | #3 | #4 | #5 | | | |
| Measured weight (mg) | 129.37 | 132.46 | 135.00 | 137.78 | 126.46 | 132.21 | 4.47 | 3.4 |
| Calculated volume (mL) | 124.63 | 127.61 | 130.06 | 132.74 | 121.83 | 127.37 | 4.31 | 3.4 |

### <Example 6>

In the present embodiment, the liquid pharmaceutical of Example 1 was filled in a plastic syringe made of a COP material by using the method of Example 2, and the main force was analyzed. The syringe pressure was analyzed into a stopper break-loose force, which is a force required to move the stopper 2 and start the dosing of an injection solution, and a stopper gliding force, which is a force required from the start of dosing of the injection solution until all the injection solutions are dosed.

Specifically, aflibercept liquid pharmaceutical having a final concentration of about 40 mg/mL was filled in a COP syringe, and the stopper break-loose force and the stopper sliding force were analyzed using a push pull gauge equipment manufactured by the Zwick company.

Five syringes and samples were temperature-equilibrated for at least 4 hours in a standard atmosphere: 23°C ± 5°C, and 50% ± 25% relative humidity. The luer lock of the temperature-equilibrated syringe was removed and mounted on a syringe holder. The "force" of the equipment was set to zero and the syringe pressure was measured.

Five syringes were set as one set, and the stopper break-loose force and the stopper sliding force for a total of five sets were tested.

A glide start position was set at 0.5 mm from a stopper break-loose force peak, and a glide end position was located before the force rises steeply at the end of the measurement. The gliding speed was performed at 100 mm/min.

The results of analyzing the stopper break-loose force are shown in Table 5.

**[Table 5]**

| No. | Break-loose force (N) | | Glide force (N) | |
|---|---|---|---|---|
| 1 | Mean | SD | Mean | SD |
| 2 | 209 | 0.21 | 2.30 | 0.16 |

### <Example 7>

In the present embodiment, the liquid pharmaceutical of Example 1 was contained as an active ingredient as biological molecular pharmaceutical, and in order to check whether the plastic syringe filled by using the method of Example 2 is stable, stability was confirmed by filling the liquid pharmaceutical into a glass syringe coated with silicone oil, a cyclic olefin polymer (COP) syringe that is not coated with silicone oil, and a cyclic olefin copolymer (COC) syringe that is not coated with silicone oil.

Specifically, the liquid pharmaceutical of Example 1 was filled into (1) a glass syringe coated with silicone oil; (2) COP syringes that are not coated with silicone oil; (3) COC syringes that are not coated with silicone oil; and were stored at 25°C for 12 months, and impurities generated over time were analyzed by size exclusion high-performance liquid chromatography (SE-HPLC).

For SE-HPLC analysis, 0.2 M potassium phosphate (pH 6.2), 0.25 M potassium chloride, and 0.05% (w/v) NaN₃ buffering solutions were flowed at the velocity of 0.5 mL/min using a TSK-gel G3000SWXL column (7.8X300 mm, TOSOH company, Japan), and the peak of the anti-VEGF-Fc fusion protein was confirmed at an absorbance of 280 nm.

Visible particles were visually counted using a white background and a fluorescent lamp according to the US Pharmacopoeia (USP <788> particulate matter in injection). Sub-visible particles were counted according to the European Pharmacopoeia (EP 2.9.19.) using a Liquid particle counter from Backman Coulter company.

In the case of sub-visible particles, 3 mL of a sample and 27 mL of particle-free water were mixed to make the total volume of 30 mL of a sample solution, and then, the container was inverted about 20 times and used. When there are fine air bubbles in the sample, it has a great effect on the measured value, and thus the container was inverted and shaken, was mixed and then was left for 10 minutes before measurement and was measured.

Specimens were collected in a 50 mL conical tube and the sensor position of a liquid particle counter was adjusted in the sample solution. The EP standard procedure was established, and the liquid particle counter was operated. The result is as follows.

During storage for 6 months, visible particles were formed in the glass pre-filled syringe. Thus, analysis of sub-visible particles was not performed. Also, in the case of the pre-filled syringe made of glass, the content of impurities was measured to be about twice higher than that of the sample stored in the pre-filled syringe made of plastic at 12 months. In the case of the plastic pre-filled syringe, the number of particles was similarly analyzed within a significant range with the time of filling, so that no particular trend could be identified.

**<Table 6>**

| | Specification | Manufacturer | | | | | | | | | | | |
|---|---|---|---|---|---|---|---|---|---|---|---|---|---|
| SE-HPLC | (DP) | Type 1 glass PFS | | | | COC PFS | | | | COP PFS | | | |
| | Total Impurity NMT 5.0% | 0 M | 3 M | 6 M | 12 M | 0 M | 3 M | 6 M | 12 M | 0 M | 3 M | 6 M | 12 M |
| | | 0.7 | 1.4 | 1.8 | 6.3 | 0.6 | 1.4 | 1.8 | 2.4 | 0.7 | 1.5 | 1.8 | 2.5 |

**<Table 7: Sub-visible particulate >**

| | | | | | | | | | | | | | |
|---|---|---|---|---|---|---|---|---|---|---|---|---|---|
| Sub-visible Particulate | Specification (DP) | Manufacturer | | | | | | | | | | | |
| | | Type 1 glass PFS | | | | COC PFS | | | | COP PFS | | | |
| | | 0 M | 3 M | 6 M | 12 M | 0 M | 3 M | 6 M | 12 M | 0 M | 3 M | 6 M | 12 M |
| | (NLT 10 pm) Particle NMT 2,000 per mL | 60 | 64 | | | 86 | 26 | 61 | 41 | 60 | 49 | 53 | 32 |
| | (NLT 25 pm) Particle NMT 50 per mL | 2 | 2 | | | 2 | 2 | 1 | 1 | 2 | 2 | 3 | 1 |

**<Table 8: Insoluble particle>**

| | | | | | | | | | | | | | |
|---|---|---|---|---|---|---|---|---|---|---|---|---|---|
| Insolubl e Particle | Specificatio n (DP) | Manufacturer | | | | | | | | | | | |
| | | Type 1 glass PFS | | | | COC PFS | | | | COP PFS | | | |
| | | 0 M | 3 M | 6 M | 12 M | 0 M | 3 M | 6 M | 12 M | 0 M | 3 M | 6 M | 12 M |
| | Essentially Free (USP 790) | Fre e | Fre e | Visible Particl e | Visible Particl e | Fre e | Fre e | Fre e | Free | Fre e | Fre e | Fre e | Free |

### (Explanation of reference numerals)

1: barrel
1a: outlet
1b: inlet
2: stopper
3: liquid pharmaceutical
4: first space
5: second space
6: tip
7: cover
t: guide tube
r: rod

## Claims

1. A pre-filled syringe comprising:
a hollow barrel comprising an outlet through which liquid pharmaceutical is discharged, and an inlet in an opposite direction to a direction of the outlet;
a stopper disposed inside the barrel; and
a plunger capable of being in contact with the stopper and moving the stopper toward the outlet to vary an internal volume of the barrel between the stopper and the outlet,
wherein the barrel has a nominal maximum filling volume of about 0.5 mL to about 1 mL, and the liquid pharmaceutical is filled inside the barrel so that the barrel has a dosing volume of about 0.03 mL to about 0.2 mL, and
after filling the liquid pharmaceutical into the barrel, a space is formed between the stopper and the surface of the liquid pharmaceutical, and a volume of the space is 0 mL to about 0.9 mL.

2. The pre-filled syringe of claim 1, wherein the volume of the space is 0mL to about 0.6 mL.

3. The pre-filled syringe of claim 1, wherein an inside of the barrel is filled with about 0.11 mL to about 0.17 mL of the liquid pharmaceutical.

4. The pre-filled syringe of claim 1, wherein a distance from the surface of the liquid pharmaceutical to the stopper, which is a height of the space, is about 10 mm or less.

5. The pre-filled syringe of claim 1, wherein the barrel has an inner diameter of about 3 mm to about 8 mm.

6. The pre-filled syringe of claim 1, wherein the liquid pharmaceutical is filled in the barrel so that the liquid pharmaceutical has a dosing volume of about 0.126 mL to about 0.154 mL.

7. The pre-filled syringe of claim 1, wherein the liquid pharmaceutical is liquid pharmaceutical containing a vascular endothelial growth factor (VEGF) antagonist solution.

8. The pre-filled syringe of claim 7, wherein the liquid pharmaceutical containing the vascular endothelial growth factor antagonist solution have a concentration of about 1 mg/mL to about 300 mg/mL.

9. The pre-filled syringe of claim 7, wherein the liquid pharmaceutical containing the vascular endothelial growth factor antagonist solution have a specific gravity of about 1 to about 1.1.

10. The pre-filled syringe of claim 7, wherein the liquid pharmaceutical containing the vascular endothelial growth factor antagonist solution have a viscosity of about 3 cP to about 4 cP.

11. The pre-filled syringe of claim 7, wherein the liquid pharmaceutical containing the vascular endothelial growth factor antagonist solution have about pH 5 to about pH 8.

12. The pre-filled syringe of claim 1, wherein the barrel is made of plastic, and silicone oil is not applied therein.

13. The pre-filled syringe of claim 1, wherein the barrel is made of cyclic olefin copolymer (COC) or cyclic olefin polymer (COP).

14. A pre-filled syringe comprising a hollow barrel having an outlet through which liquid pharmaceutical is discharged, and an inlet in an opposite direction to the outlet, a stopper disposed inside the barrel, and a plunger capable of being in contact with the stopper and moving the stopper toward the outlet to vary an internal volume of the barrel between the stopper and the outlet, wherein the pre-filled syringe is manufactured by performing operations of:
filling liquid pharmaceutical inside of the barrel;
combining a vacuum chamber with the barrel and applying an initial vacuum pressure to the inside of the barrel;
applying a process vacuum pressure lower than the initial vacuum pressure to the inside of the barrel and disposing the stopper inside of the barrel; and
releasing the process vacuum pressure applied to the inside of the barrel.

15. The pre-filled syringe of claim 14, wherein, after the filling of the liquid pharmaceutical inside of the barrel, the operations further comprise forming a first space between the outlet and the liquid pharmaceutical.

16. The pre-filled syringe of claim 15, wherein, after the applying of the initial vacuum pressure to the inside of the barrel, the operations further comprise moving air in the first space toward the inlet.

17. The pre-filled syringe of claim 16, wherein, after the disposing of the stopper inside the barrel, the operations further comprise forming a second space between the stopper and the liquid pharmaceutical, wherein the height of the second space is less than the height between the stopper and the inlet.

18. The pre-filled syringe of claim 17, wherein, after the releasing of the process vacuum pressure applied to the inside of the barrel, the operations further comprise moving the stopper up to a certain position toward the liquid pharmaceutical.

19. The pre-filled syringe of claim 18, wherein the barrel has a nominal maximum fill volume of about 0.5 mL to about 1 mL, and is filled with liquid pharmaceutical inside the barrel to have a dosing volume of about 0.03 mL to about 0.2 mL, and after the moving of the stopper to a predetermined position toward the liquid pharmaceutical, the volume of the space formed between the liquid pharmaceutical and the stopper is 0 mL to about 0.9 mL.
